(19)

Europäisches
Patentamt
European
Patent Office
Office européen
des brevets

(11) **EP 4 527 920 A1**

(12) **EUROPEAN PATENT APPLICATION**

(43) Date of publication:
**26.03.2025 Bulletin 2025/13**

(21) Application number: **24194353.9**

(22) Date of filing: **13.08.2024**

(51) International Patent Classification (IPC):
*C12N 5/00* (2006.01)

(52) Cooperative Patent Classification (CPC):
**C12N 5/0068;** A61L 27/20; A61L 27/56;
C12M 25/14; C12N 2513/00; C12N 2533/74;
C12N 2533/78

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.08.2023 US 202363520352 P**

(71) Applicant: **Gelatex Technologies OÜ**
**13811 Tallinn (EE)**

(72) Inventors:
• **Banerjee, Arindam**
**711227 Howrah (IN)**
• **Martens, Märt-Erik**
**75312 Rae vald (EE)**

• **Liira, Rasmus**
**11411 Tallinn (EE)**
• **Neivelt, Karl Indrek**
**10111 Tallinn (EE)**
• **Mölder, Lauri**
**11619 Tallinn (EE)**
• **Howlader, Md Musa**
**10132 Tallinn (EE)**
• **Loos, Magnus**
**13620 Tallinn (EE)**
• **Enn, Raido**
**75304 Järveküla (EE)**

(74) Representative: **Moosedog Oy**
**Vähäheikkiläntie 56C**
**20810 Turku (FI)**

(54) **PLANT-BASED MICROFIBROUS SCAFFOLDS FOR CULTURING MEAT**

(57)    The present disclosure relates to plant-based microfibrous scaffolds and a method for producing these scaffolds for culturing meat, providing a biocompatible, edible, and scalable 3D structure that supports high cell yield. The scaffold comprises plant-based proteins, polysaccharides, and carbohydrates, eliminating the need for synthetic polymers and toxic solvents. The method for producing these scaffolds comprises dissolving the components, creating a homogeneous solution, spinning the fibers using air volume and centrifugal forces, and heating to achieve crosslinking. The resulting scaffolds have controlled fiber diameters, thicknesses, and area densities, enhancing cell growth, nutrient diffusion, and structural integrity and provide an efficient and sustainable solution for large-scale cultured meat production.

FIG. 1

EP 4 527 920 A1

**Description**

TECHNICAL FIELD

The present disclosure relates generally to tissue engineering technologies; and more specifically, to plant-based microfibrous scaffolds and methods for producing such scaffolds for culturing meat.

BACKGROUND

In recent years, there has been a growing interest in food products that offer a meat-like eating experience and nutritional value without the environmental risks and ethical concerns associated with animal-based meat. The concept of lab-grown meat (also known as cultured meat, clean meat, cell-based meat), originating from tissue engineering applications, aims to provide a sustainable and ethical alternative to conventionally slaughtered meat. To successfully replace traditional meat and meet consumer demands, cultured meat technologies require significant improvements in scaffold design, ensuring 3-dimensional structure, high yield of cell culture, biocompatible, reproducible, edible, scalable, and cost-effective.

Plant-derived materials, such as proteins, polysaccharides, and carbohydrates, have emerged as a crucial solution for scaffold production due to their cost-effectiveness, wide availability, edibility, and biocompatibility, making them attractive for large-scale applications. The utilization of plant-based materials for meat production has been investigated with a wide variety of plant extracts and different technologies. However, there are challenges in creating a porous 3D structure that could increase the yield of cells thereby facilitating better organoleptic properties in the final product.

Various methods have been explored to produce scaffolds for cultured meat, with electrospinning being the predominant method. This method involves creating nanofibers from polymer solutions resulting in a high surface area to promote cell attachment, growth and yield of cells, biomimetic resemblance to natural extracellular matrix, and tunable mechanical properties for desired texture. The electrospinning technology was attempted to create scaffolds for cultured meat that is made of plant-based materials and synthetic polymers as a carrier-agent such as PEO, PDGL, and PCL (International Patent Application No. PCT/US2020/016368). These synthetic polymers can be challenging for the human digestive system to break down, potentially leading to digestive issues or incomplete nutrient absorption. In another patent application (International Patent Application No. PCT/US2019/066089), plant-based fibrous scaffolds have been developed with a wide range of plant-derived materials and animal polymers via electrospinning and solution blow spinning. The inventors stated that the spun solution was prepared in volatile solvent systems that may include acetone, ethyl acetate, methanol, phenol, and dimethylformamide. These solvents might have detrimental effects due to their toxicity, potential residue presence, lack of biocompatibility with cells, negative environmental impact, and regulatory restrictions in food production.

As cultured meat industries move towards large-scale production, there is a clear and urgent demand for improved plant-based scaffolds, devoid of animal-protein, synthetic polymers, and toxic solvents. These advancements are essential to enhance production in a cost-effective manner and at a significant scale, while ensuring the viability of the process. Scalable scaffold techniques play a vital role in meeting these requirements and supporting the successful development of culturing meat.

In another example, the HaloSpin™ apparatus and method for producing polymer fibers by Gelatex Technology (US Patent Grant Number 11697892) offers production of microfibrous scaffold. Although it thereby overcoming many challenges, and meeting the demand of producing plant-based scaffolds in large-scale for cultured meat industries it doesn't enable the assembly of fibers into uniform 3D scaffolds, which affects the structure's internal architecture and porosity, which is crucial for cell growth. Additionally, the process relies on synthetic polymers and toxic solvents, making it difficult to ensure the final product is free from harmful residues. The method also lacks the ability to precisely control fiber alignment and distribution, which is important for replicating the extracellular matrix and supporting high cell yield. Furthermore, the scalability of the process to produce large, consistent 3D scaffolds remains unproven, posing challenges for mass production. Further, the mechanical properties of the fibers produced may not be suitable for the dynamic and varied mechanical environment required for different tissue types.

SUMMARY

The aim of the present disclosure is to provide a biocompatible, edible, and scalable 3D scaffold structure that supports high cell yield and is free from synthetic polymers and toxic solvents. The aim of the disclosure is achieved by a cell culture scaffold and a method for making a cell culture scaffold as defined in the appended independent claims to which reference is made to. Advantageous features are set out in the appended dependent claims.

The advantages of the present disclosure are sustainability and environmental benefits due to the use of plant-based materials, reduced allergenic potential, and the presence of bioactive compounds enhancing health benefits. The technology allows for cost-effective, scalable production with tailorable mechanical properties and improved cell growth and differentiation. Furthermore, the process ensures regulatory compliance by eliminating toxic solvents and synthetic polymers, making the scaffolds safe and edible for cultured meat applications. This innovation highlights the significance of plant-based materials in microfibrous scaffold development, contributing to a sustainable and ethical alternative to traditional meat.

The cell culture scaffold according to the present disclosure ensures uniform fiber distribution and porosity, enhance cell attachment, growth, and differentiation, and is for consumption, thereby addressing the current challenges in producing cultured meat at a large scale in a cost-effective and environmentally friendly manner. It thus provides a an important alternative to traditional meat production, supporting the growing demand for sustainable and ethical food sources.

Additional aspects, advantages, features and objects of the present disclosure would be made apparent from the drawings and the detailed description of the illustrative embodiments construed in conjunction with the appended claims that follow.

BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the embodiments of the disclosure are shown in the drawings, with references to the following diagrams wherein:

Figure 1, presents a section of plant-based scaffold showing microfibers embedded in a thickness 500 $\mu$m.

Figure 2a, presents a SEM image of microfibrous scaffolds (Magnification 500X) consisting of potato protein isolate (60%), pullulan (32%), and dextrose (8%).

Figure 2b, presents a SEM image of microfibrous scaffolds (Magnification 7000X) consisting of potato protein isolate (60%), pullulan (32%), and dextrose (6%).

Figure 3a, presents a SEM image of microfibrous scaffolds (Magnification 500X) consisting of Chitosan from mushroom (66%), lupin protein isolate (14%), pullulan (15%), and dextrose (5%).

Figure 3b, presents a SEM image of microfibrous scaffolds (Magnification 5000X) consisting of Chitosan from mushroom (66%), lupin protein isolate (14%), pullulan (15%), and dextrose (5%).

Figure 4a, presents a SEM image of microfibrous scaffolds (Magnification 500X) consisting of soy protein isolate (66%), potato protein isolate (14%), pullulan (16%), and glucose (4%).

Figure 4b, presents a SEM image of microfibrous scaffolds (Magnification 7000X) consisting of soy protein isolate (66%), potato protein isolate (14%), pullulan (16%), and glucose (4%).

Figure 5, shows a continuous production of microfibrous scaffold in HaloSpin™ apparatus.

Figure 6, shows C2C12 cells after proliferating for 7 days in Potato protein-based scaffold.

Figure 7, shows C2C12 cells after proliferating for 7 days in chitosan-lupin-based scaffold.

DETAILED DESCRIPTION OF EMBODIMENTS

The following detailed description illustrates embodiments of the present disclosure and ways in which they can be implemented. The present disclosure provides a cell culture scaffold and a method for making a cell culture scaffold. The present disclosure significantly improves the methods and materials for producing plant-based microfibrous scaffolds used in culturing meat by offering a biocompatible, edible, and scalable 3D structure that supports high cell yield. This eliminates the need for synthetic polymers and toxic solvents, thereby enhancing the safety, sustainability, and regulatory compliance of the final product. Additionally, it allows for precise control over fiber diameter and scaffold thickness, ensuring highly porous and optimal mechanical properties, which are crucial for effective cell growth and differentiation.

In an aspect, the present disclosure provides a cell culture scaffold comprising plant-based microfibrous in a 3D structure, wherein the plant-based microfibrous comprise at least 60% of one or more plant-based proteins, one or more polysaccharides, one or more carbohydrates. These features address the technical problem of developing a biocompatible, edible, and scalable 3D scaffold structure that supports high cell yield for culturing meat without using synthetic polymers or toxic solvents. In the context of the present disclosure, a "3D structure" refers to a scaffold with three-dimensional characteristics, including interconnected microfibers forming a network that mimics the natural extracellular matrix. This structure supports cell attachment, growth, and differentiation, allowing efficient nutrient diffusion and waste removal, essential for cultured meat applications.

The use of plant-based proteins ensures that the scaffold is derived from renewable and sustainable sources, reducing the environmental impact and making the scaffold safe for consumption. The polysaccharides contribute to the mechanical stability and structural integrity of the scaffold, enhancing its ability to support cell attachment, growth, and differentiation. The inclusion of carbohydrates aids in the crosslinking process, providing additional strength and stability to the scaffold. The combination of these components results in a scaffold that mimics the natural extracellular matrix, promoting efficient nutrient diffusion, waste removal, and cell interactions, ultimately leading to improved cell proliferation and tissue formation. This ensures that the scaffold is both scalable and cost-effective, meeting the demands for large-scale production in cultured meat applications.

According to the embodiments of the present disclosure, the fibrous scaffold architecture creates a 3D network resembling a sheet-like, entangled fibers in a single embodiment, allowing cell interactions, nutrient diffusion, and waste removal. Adjusting process parameters enables better control over fiber diameter and scaffold thickness, enhancing uniform porosities and continuous fiber orientation and entanglement.

In another aspect, the present disclosure provides a method for making a cell culture scaffold comprising plant-based microfibrous in a 3D structure, wherein the method comprises dissolving one or more plant-based proteins in a solvent; adding one or more polysaccharides and one or more carbohydrates until the solution becomes homogeneous; spinning the plant-based microfibrous from the homogeneous solution by a combined forces of air volume and centrifugal forces pushing the homogeneous solution out from one or more spinning needles; obtaining the cell culture scaffold from the spun plant-based microfibrous by collecting the spun plant-based microfibrous on a substrate; heating the obtained cell culture scaffold. The method provides a scalable and efficient process for creating biocompatible and edible 3D scaffolds without synthetic polymers or toxic solvents. The dissolution and homogenization steps ensure uniform distribution of components, critical for consistent fiber formation. The spinning process, driven by air and centrifugal forces, enables the creation of microfibers with controlled diameter and orientation, mimicking the natural extracellular matrix. Collecting the fibers on a substrate forms the 3D network necessary for cell growth, while the heating step crosslinks the scaffold, enhancing its mechanical properties and stability. This method ensures high cell yield, scalability, and cost-effectiveness, making it suitable for large-scale cultured meat production.

According to the embodiments of the present disclosure, the homogeneous solution comprises 8% w/w (percent weight by weight) - 26 % w/w (percent weight by weight) of one or more plant based proteins, 5% w/w - 15% w/w of one or more polysaccharides, 1% w/w - 5% w/w of one or more carbohydrates. Thus, the homogeneous solution may comprise one or more plant-based proteins from 8, 9, 10, 11, 12, 13, 14, 15, 16, 17, 18, 19, 20, 21, 22, 23, 24, or 25 % w/w up to 9, 10, 11, 12, 13, 14, 15, 16, 17, 18, 19, 20, 21, 22, 23, 24, 25 or 26 % w/w of one or more plant-based proteins; one or more polysaccharides from 5, 6, 7, 8, 9, 10, 11, 12, 13, or 14 % w/w up to 6, 7, 8, 9, 10, 11, 12, 13, 14 or 15% w/w of one or more polysaccharides; one or more carbohydrates from 1, 2, 3, or 4% w/w up to 2, 3, 4, or 5% w/w of one or more carbohydrates. Such composition ensures an optimal balance of components for fiber formation, mechanical strength, and biocompatibility. The specified range of plant-based proteins provides sufficient structural integrity and nutritional value, while the polysaccharides contribute to the scaffold's mechanical properties and spinnability. The carbohydrates aid in the crosslinking process, enhancing the stability and durability of the scaffold. The precise ratios ensure a homogeneous solution, which is crucial for consistent fiber formation and uniform scaffold architecture. Thes leads to a scaffold that mimics the natural extracellular matrix, supports high cell yield, and is suitable for large-scale production of cultured meat, while being cost-effective, biocompatible, and free from synthetic polymers and toxic solvents.

According to the embodiments, the parameters of spinning are: an air volume 20 m3/hour - 35 m3/hour, an air temperature 30 °C - 80 °C, solution pressure 0.1 bar - 1.5 bar, wherein the substrate is rotating at a speed rate of 2.3 m/hour - 250 m/hour. The parameters of spinning can thus be as follows. The air volume can be from 20, 21, 22, 23, 24, 25, 26, 27, 28, 29, 30, 31, 32, 33, or 34 m$^3$/hour up to 21, 22, 23, 24, 25, 26, 27, 28, 29, 30, 31, 32, 33, 34, or 35 m$^3$/hour. The air temperature can be from 30, 35, 40, 45, 50, 55, 60, 65, 70, or 75 °C up to 35, 40, 45, 50, 55, 60, 65, 70, 75, or 80 °C. The solution pressure can be from 0.1, 0.2, 0.3, 0.4, 0.5, 0.6, 0.7, 0.8, 0.9, 1.0, 1.1, 1.2, 1.3, or 1.4 bar up to 0.2, 0.3, 0.4, 0.5, 0.6, 0.7, 0.8, 0.9, 1.0, 1.1, 1.2, 1.3, 1.4, or 1.5 bar. The substrate rotation speed can be from 2.3, 5, 10, 20, 30, 40, 50, 60, 70, 80, 90, 100, 120, 140, 160, 180, 200, 220, or 240 m/hour up to 5, 10, 20, 30, 40, 50, 60, 70, 80, 90, 100, 120, 140, 160, 180, 200, 220, 240, or 250 m/hour. These parameters enable to provide precise control over the fiber formation process, ensuring uniformity and consistency in the scaffold structure. The controlled air volume and temperature facilitate the efficient evaporation of the solvent, aiding in the formation of continuous and stable microfibers. The solution pressure ensures the homogeneous solution is extruded at a consistent rate, crucial for maintaining uniform fiber diameter. The adjustable substrate rotation speed allows for fine-tuning the fiber deposition, resulting in a well-organized 3D network. This leads to the production of high-quality scaffolds with optimal porosity and mechanical properties, enhancing cell attachment, growth, and differentiation, thereby supporting large-scale, cost-effective cultured meat production.

According to the embodiments, the heating of the obtained cell culture scaffold is performed by keeping the obtained cell culture scaffold in a hot air at 121 °C - 200 °C for 2 - 120 minutes. The parameters of the heating of the obtained cell culture scaffold can thus be as follows. The hot air temperature can be from 121, 130, 140, 150, 160, 170, 180, or 190 °C up to 130, 140, 150, 160, 170, 180, 190, or 200 °C. The heating duration can be from 2, 5, 10, 20, 30, 40, 50, 60, 70, 80, 90, 100, or 110 minutes up to 5, 10, 20, 30, 40, 50, 60, 70, 80, 90, 100, 110, or 120 minutes. This heating process ensures the crosslinking of the plant-based proteins and other components within the scaffold. The temperature range and duration are crucial for achieving optimal crosslinking without degrading the material, thereby enhancing the scaffold's mechanical strength, stability, and durability. The optimal crosslinking improves structural integrity and resilience of the scaffold, which are essential for maintaining its 3D architecture during cell culture. Additionally, this controlled heating process ensures the scaffold remains biocompatible and edible, making it suitable for large-scale production of cultured meat while ensuring the safety and quality of the final product.

The current disclosure introduces the production of microfibrous scaffolds that may serve both as a source of protein-rich food and as a scaffold for cultivated meat applications. These microfibrous scaffolds possess several novel features that set them apart, including edibility, the presence of plant material, a 3-dimensional structure, unique morphology, and tunable properties, which distinguish them from both solution blow spinning and electrospinning techniques. The novel scaffolds are produced by HaloSpin™ (US Patent Grant Number 11697892) apparatus. The fibrous architecture of the scaffold creates a porous, interconnected 3D network, and mimics the natural extracellular matrix of tissues and facilitates cell interactions, nutrient diffusion, and waste removal.

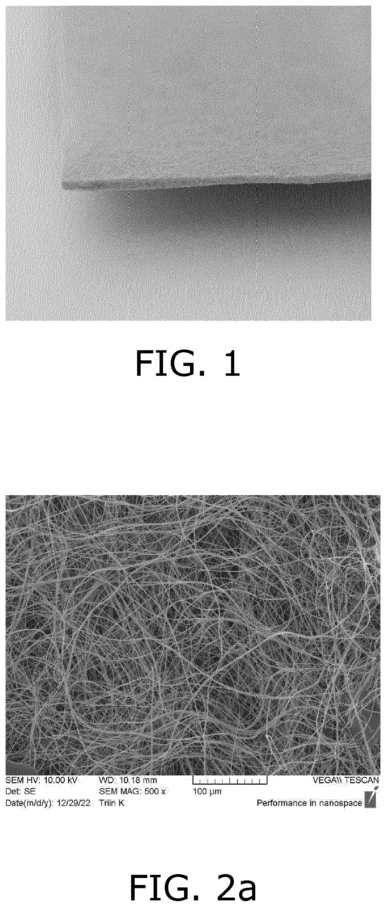

In most embodiments, the fibrous structure of any individual scaffold resembles a 3D network of entangled fibers, a narrow distribution of fiber diameter, appropriate thickness of the scaffold, and fiber orientation such as random or aligned. The distinct structure of the scaffolds resembles a felt-like sheet (Figure 1).

In certain embodiments, the process parameters from HaloSpin™ apparatus may be adjusted to obtain different fiber diameter thereby, offering better control over scaffold thickness that resulting in more uniform porosities, and 3D architecture with continuous fiber entanglement along increment of the thickness.

In certain embodiments, the continuous production of scaffolds involves adjusting various effective parameters for different polymer recipes. These parameters include solution composition, solution concentration, solution pressure, solution temperature, air volume, air temperature, the number of needles, needle sizes, and belt speed (collection of fibers), either individually or in combination. Such parameter adjustments not only accelerate the process but also enable easier scalability for mass production compared to electrospinning.

In an embodiment, a microfibrous scaffold comprises one or more plant proteins, one or more polysaccharide, and one or more carbohydrate.

In an embodiment, the scaffolds are produced in three steps including preparation of recipe, spinning in HaloSpin™ apparatus, and thermally crosslinking the scaffolds.

In an embodiment, the term "recipe" refers to the final solution prepared for spinning, containing one or more plant proteins, polysaccharide, and carbohydrate in different weight percent.

In an embodiment, the term "spun", "spinning", and "spin" refers to the solution spun in a HaloSpin™ apparatus by Gelatex.

In an embodiment, the term "curing", "ovening", "heating" refers to crosslinking the plant proteins by applying thermal heat on the scaffolds at a certain temperature and time.

Step 1: Preparation of final recipe

In certain embodiments, the plant protein in the scaffold is either protein isolates, protein concentrates, protein hydrolysate, peptides, or any other form of plant proteins. Example, in general a protein isolate is > 70% protein and protein concentrate is < 50% protein, based on individual protein profile scaffold goals can meet up the desired nutritional profile in a product.

In certain embodiments, the source of plant protein includes but is not limited to potato, soy, pea, lupin, canola, fava, mung, corn, wheat, rice, barley, sunflower, or derivatives and/or combinations thereof.

In certain embodiments of the cell culture scaffold, the polysaccharide includes but is not limited to pullulan, starch, pectin, alginate, chitosan or combinations or derivatives thereof.

In certain embodiments, the pullulan is a crucial component determining the spinnability of a recipe.

In certain embodiments of the cell culture scaffold, the carbohydrate includes reducing sugar or sugar alcohols including, but not limiting, dextrose, fructose, galactose, glyceraldehyde, glucose, isomalt, lactitol, maltose, mannitol, maltitol, ribose, sorbitol, xylitol, xylose or combinations thereof.

In certain embodiments, the solvents used in dissolving the proteins, polysaccharide, and carbohydrate include water, alcohols, aqueous salts, acetic acid, and formic acid or combinations, therefore. The aqueous salts solutions are prepared from salts including $NaCl$, $NaHCO_3$, $Na_2CO_3$, $KCl$, $CaCl_2$, $MgCl_2$, $Na_2HPO_4$, $KH_2PO_4$, and $MgSO_4$.

In certain embodiments, the concentration of a plant protein is in the range of 8 wt% to 26 wt% of the final recipe.

In certain embodiments, the concentration of a polysaccharide is in the range of 5 wt% to 15 wt% of the final recipe.

In certain embodiments, the concentration of a carbohydrate is in the range of 1 wt% to 5 wt% of the final recipe.

In certain embodiments, a small percentage of additive agents are added in the final recipe to enhance the scaffold properties.

In some cases, the carbohydrates can be used either to crosslink the plant protein and/or present in the scaffolds as sweetening agents that provide flavor.

Step 2: Spinning of final recipe.

In an embodiment, the final recipe is spun in a HaloSpin™ apparatus to produce a microfibrous scaffold by adjusting the parameters.

In certain embodiments, the composition of a microfibrous scaffold is 60% to 85% plant protein, 10% to 20% polysaccharide, 1% to 10% carbohydrate, or any percentages in-between.

In certain embodiments, the composition of plant proteins in a microfibrous scaffold may contain one or more proteins.

In certain embodiments, the composition of plant proteins in a microfibrous scaffold may contain one or more polysaccharides.

In certain embodiments, the composition of plant proteins in a microfibrous scaffold may contain one or more carbohydrates.

In certain embodiments, the fiber diameter may be tuned in-between 0.3 $\mu$m to 3 $\mu$m, by changing the concentration of the final solution or by changing the process parameter or combinations thereof. More specifically, according to the embodiments of the present disclosure, the diameter of the plant-based microfibrous may be 0.3 $\mu$m - 3 $\mu$m. The diameter of the plant-based microfibrous can thus be from 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.2, 2.2, 2.3, 2.4, 2.5, 2.6, 2.7, 2.8 or 2.9 $\mu$m up to 0.4, 0.5, 0.6, 0.7, 0.8, 0.9, 1.0, 1.1, 1.2, 1.3, 1.4, 1.5, 1.6, 1.7, 1.8, 1.9, 2.0, 2.2, 2.2, 2.3, 2.4, 2.5, 2.6, 2.7, 2.8, 2.9 or 3.9 $\mu$m. The diameter of plant-based microfibrous in this range enables closely to mimic the natural extracellular matrix, which is essential for supporting cell attachment, growth, and differentiation. The diameter of the microfibers increases the surface area available for cell interaction, enhancing cell adhesion and proliferation. Additionally, this range allows for creating a porous scaffold structure that facilitates efficient nutrient diffusion and waste removal. This provides improved cell yield and tissue formation, as well as the creation of a more physiologically relevant environment for cultured meat production. This control over fiber diameter ensures the scaffold meets the necessary mechanical and biological requirements for effective large-scale cultured meat applications.

In certain embodiments, the scaffold thickness may be tuned in-between 50 $\mu$m to 3 cm, by changing the concentration of the final solution or by changing the process parameter or combinations thereof. More specifically, according to the embodiments of the present disclosure, the thickness of the cell culture scaffold may be 50 $\mu$m - 30 000 $\mu$m. The thickness of the cell culture scaffold can thus be from 50, 60, 70, 80, 90, 100, 110, 120, 130, 140, 150, 160, 170, 180, 190, 200, 220, 240, 260, 280, 300, 350, 400, 450, 500, 550, 600, 650, 700, 750, 800, 850, 900, 950, 1000, 1100, 1200, 1300, 1400, 1500, 1600, 1700, 1800, 1900, 2000, 2200, 2400, 2600, 2800, 3000, 3500, 4000, 4500, 5000, 5500, 6000, 6500, 7000, 7500, 8000, 8500, 9000, 9500, 10000, 11000, 12000, 13000, 14000, 15000, 16000, 17000, 18000, 19000, 20000, 22000, 24000, 26000 or 28000 $\mu$m up to 60, 70, 80, 90, 100, 110, 120, 130, 140, 150, 160, 170, 180, 190, 200, 220, 240, 260, 280, 300, 350, 400, 450, 500, 550, 600, 650, 700, 750, 800, 850, 900, 950, 1000, 1100, 1200, 1300, 1400, 1500, 1600, 1700, 1800, 1900, 2000, 2200, 2400, 2600, 2800, 3000, 3500, 4000, 4500, 5000, 5500, 6000, 6500, 7000, 7500, 8000, 8500, 9000, 9500, 10000, 11000, 12000, 13000, 14000, 15000, 16000, 17000, 18000, 19000, 20000, 22000, 24000, 26000, 28000 or 30000 $\mu$m.

This range of scaffold thickness allows customization of the scaffold to suit different cell culture requirements and applications in cultured meat production. Thinner scaffolds (e.g.,50-1000 $\mu$m) are suitable for applications needing rapid nutrient diffusion and waste removal, while thicker scaffolds (up to 30,000 $\mu$m) provide greater structural support and are suitable for more complex tissue structures. This provides the ability to tailor the scaffold thickness to optimize cell growth conditions, improve tissue integrity, and accommodate various types of cells and tissue constructs. This flexibility ensures that the scaffold can support high cell yields and efficient tissue maturation, making it suitable for scalable and diverse cultured meat production applications.

For example, the thickness of the cell culture scaffold may be 50 $\mu$m - 100 $\mu$m, 200 $\mu$m - 500 $\mu$m, or 1000 $\mu$m - 5000 $\mu$m. The cell culture scaffolds in 50 $\mu$m - 100 $\mu$m range enable to achieve enhanced nutrient diffusion and waste removal, thus offering minimal diffusion distances and ensuring that cells receive adequate nutrients and oxygen while efficiently removing metabolic waste. This is particularly important for high-density cell cultures, where maintaining a healthy microenvironment is crucial. Improved structural integrity for complex tissues is achieved with the 200 $\mu$m - 500 $\mu$m range, which balances nutrient diffusion with structural support, making it suitable for more complex tissues that require both. This range can support multilayered cell structures while still allowing sufficient nutrient transport. Cell culture scaffolds in the 1000 $\mu$m - 5000 $\mu$m range provide the necessary mechanical strength and structural integrity for larger and more complex tissue constructs. This range is particularly advantageous for developing thicker tissue slices or whole muscle structures, where mechanical stability is crucial for maintaining tissue architecture during growth and differentiation. The ability to precisely control scaffold thickness within these narrow ranges allows for the creation of customized microenvironments that can be optimized for different cell types and tissue engineering applications. This level of control is unexpected and advantageous, as it enables researchers and producers to fine-tune the scaffolds for specific cell behaviors and outcomes.

Step 3: Thermal heating the scaffolds for crosslinking.

In an embodiment, the scaffolds after production are then crosslinked by putting the scaffolds in an oven at a range of 121 °C to 200 °C temperature or any temperature in-between. The duration of curing may vary in the range of 2 minutes to 2 hours or any time in-between.

In certain embodiments, the amount of scaffold produced in the HaloSpin™ apparatus is 0.1 kg/hour - 25 kg/hour. The continuous spinning of a recipe may extend until 20 days to 30 days of operation.

Disclosed herein, the production of an edible microfibrous scaffold for cultured meat applications. The morphology of the scaffolds (Figure 2a, 3a, 4a) showed the microfibrous architecture that closely resembles the natural ECM.

The randomly oriented fibers are fitted in a single embodiment of the scaffold. The SEM images (Figure 2a, 2b, 3a, 3b, 4a, 4b) also showed fiber entanglement, high porosity, high surface to volume ratio, and free passages/pathways for the sufficient absorption of nutrients as well as transport/exchange of the same.

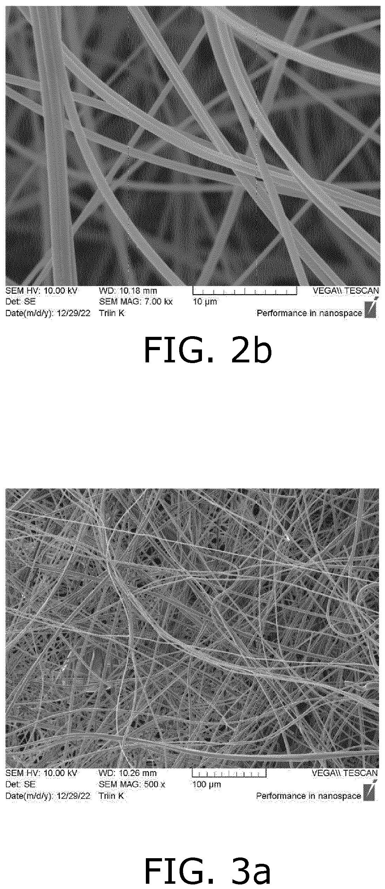

The free pathways are due to the gaps in-between the entangled fibers that help in forming a 3D-interconnecting network throughout the scaffolds. The interconnectivity of a microfibrous scaffold is not easily visible in 2D images via SEM. However, the presence of the gaps in the adjacent planes of the SEM image suggested the connection in-between the layers (Figure 2b, 3b, 4b).

The sizes and entanglement of the fibers further enhance cellular activity, including cell adhesion, proliferation, differentiation, and migration, essential processes for tissue development and regeneration.

The macrofiber scaffold is able to retain hydration, maintaining its structural integrity and providing a supportive environment for cell growth and tissue maturation. Overall, these properties make microfibrous scaffolds well-suited for 3D cell culture and the successful maturation of tissues in tissue engineering endeavors such as culturing meat.

All the plant-based proteins and fermented products stated in the present disclosure are recognized as food grade, edible, and non-GMO. The protein isolates and protein concentrate used in this method according to the disclosure consist of >70% protein and < 50% protein respectively.

The microfiber scaffolds consist of one or more protein, one or more polysaccharide, and one or more carbohydrate.

The final recipe used for spinning typically comprises plant proteins, polysaccharide, and carbohydrate. The one or more plant-based proteins that are considered, according to the embodiments of the present disclosure, may be selected from a plant protein group comprising potato protein, soy protein, pea protein, lupin protein, canola protein, fava protein, mung protein, corn protein, wheat protein, rice protein, barley protein, or sunflower protein, or combinations or derivatives thereof.

The presence of plant proteins in scaffolds offers several advantages. They are derived from renewable sources; they are environmentally friendly and sustainable. With lower allergenic potential than animal-derived proteins, they are safer for sensitive consumers. Moreover, the lower risk of zoonotic diseases makes them ideal for cultured meat applications.

Plant proteins' large-scale availability makes them cost-effective, facilitating scalable and mass production of scaffolds.

These plant proteins contain various bioactive compounds, including isoflavones, phytosterols, flavonoids, and tocopherols, which may influence cell behavior. These compounds have been associated with antioxidant, anti-inflammatory, and antimicrobial effects, potentially impacting cell proliferation, differentiation, and signaling pathways in cultured meat applications. Therefore, the scaffold resembling the extracellular matrix, enhances cell adhesion, proliferation, and differentiation.

Altogether, plant proteins' interaction with biological processes enhances scaffold biocompatibility, making them promising candidates for cultured meat applications.

The one or more polysaccharides used along with the plant-based proteins for spinning may be selected from a group comprising pullulan, starch, pectin, alginate, chitosan, mushroom-chitosan or combinations or derivatives thereof.

The polysaccharides offer several benefits in scaffolds for culturing meat. These natural polymers are biocompatible, biodegradable, and contribute to stable and porous scaffold structures. In combination with plant proteins, they provide mechanical support, facilitate cell attachment, proliferation, and differentiation, creating a suitable microenvironment for cultured meat development.

Additionally, the presence of bioactive compounds in these scaffolds, such as polysaccharides, isoflavones, and chitosan's antimicrobial properties, may influence cell compatibility and viability. These compounds contribute to the structural strength of the scaffold, ensuring proper support for cell growth and organization.

The one or more polysaccharides may function as plant-based carrier polymers. For example, in the final recipe, pullulan might be present as a carrier polymer. This carrier polymer helps to improve the processability and spinnability of the nonspinnable polymers, allowing for the fabrication of fibers that would otherwise be challenging to produce.

The carbohydrates added may be combinations of reducing sugars or sugar alcohols, more specifically, the one or more carbohydrates may be selected from a carbohydrate group comprising dextrose, fructose, galactose, glyceraldehyde, glucose, isomalt, lactitol, maltose, mannitol, maltitol, ribose, sorbitol, xylitol, or xylose or combinations thereof.

Carbohydrates have a critical role in crosslinking proteins in the scaffolds. A crosslinking reaction occurs between carbohydrates and amino groups in proteins. It is a chemical reaction that takes place when proteins and carbohydrates are heated together, leading to the formation of insoluble scaffolds.

In some cases, the carbohydrates can be used either to crosslink the protein isolates and/or present in the scaffolds as sweetening agents that provide flavor.

The crosslinking degree of a microfibrous scaffold may be adjusted to control the desired degradation rate of the material. The crosslinking degree can be adjusted by varying the temperature and/or heating time. Also this will affect the other properties such as tensile strength, elongation, flexibility, color, smell, taste, etc.

The degradation rate of the microfibrous scaffolds may be customized to meet specific needs in culturing cells by varying the concentration of crosslinking agent in the final recipe, modifying the reaction time and temperature, or using a combination of different crosslinking agents.

The scaffolds may also typically include some additive agents that facilitates: texture, flavor, thickening, bulking, antimicrobial, preservative, pH modulator, coloring, or any combination thereof.

Additive polymers may be exemplified as modified starch, guar gum, xanthate gum, arabic gum, gellan gum, hemicellulose, gelatin, collagen, citrus fiber, plant-derived dietary fibers, yeast protein extract or any combination thereof.

The amount of additive in the final recipe is in the range of 1% (w/w) to 5% (w/w).

The solvents used for these systems are typically water, formic acid, acetic acid, alcohols, ionic compounds, or combinations thereof.

Production of scaffolds from HaloSpin™ apparatus for producing polymer fibers

The HaloSpin™ (US Patent Grant Number 11697892) apparatus utilized here for spinning a final recipe into microfibrous scaffold. HaloSpin™ technology is used since it is affordable, simple to process, and doesn't require high voltages. Additionally, this process produces fibers orders of magnitude faster than commercially available spinning methods. The detailed description of the HaloSpin™ technology is described in US Patent Grant Number 11697892.

A brief description of spinning process is illustrating as follows

The polymer solution is pushed through the needles of the apparatus for producing polymer fibers by a pressure driven flow unit and then using air flow the needles are subjected to centrifugal force either at room temperature or applying heat ranging from 30 °C to 80 °C.

The combined forces acting due to solution pressure, shear forces, and centrifugal forces resulted in pushing the fibers from the needles that travel a working distance and then collected on the substrate.

During this process the solvent quickly evaporates, and microfibers entangle upon collection to form scaffolds. In some cases, the amount of solvent present in the fiber may be no more than 0.5% (w/w) to 0.1% (w/w).

The spinneret can be positioned in different ways to obtain microfibrous in either random or aligned orientation in the scaffold.

The process parameters include solution composition, solution concentration, solution pressure, solution temperature, air volume, air temperature, the number of needles, needle sizes, spinneret design, ambient temperature and humidity and collection speed (substrate collecting fibers).

The process of preparing final recipe and spinning are as follows:

The plant proteins are first dissolved in solvent and kept stirring. Then polysaccharide and carbohydrates are added until the solution becomes homogeneous and spun in the HaloSpin™ apparatus for producing polymer fibers.

In some cases, proteins are dissolved at elevated temperatures ranging from 40°C to 80°C until the polymers dissolve completely.

In some cases, the plant proteins are either one or more in a final recipe. An example such as, potato protein isolate can be used alone or along with soy protein isolate.

In some cases, the polysaccharides are either one or more in a final recipe. An example such as, pullulan can be used alone or along with chitosan.

In some cases, the carbohydrates are either one or more in a final recipe. An example such as, glucose can be used alone or along with ribose.

In some cases, the proteins, polysaccharides, and carbohydrates are dissolved in one or more solvents or combinations thereof. For example, formic acid can be used alone or along with acetic acid for producing a chitosan-lupin scaffold, water can be used alone for producing potato scaffold.

In some cases, the concentrations of the overall proteins in a final recipe is 8% (w/w), 9% (w/w), 10 % (w/w), 11% (w/w), 12%(w/w), 13%(w/w), 14 % (w/w), 15% (w/w), 16%(w/w), 17%(w/w), 18% (w/w), 19 % (w/w), 20% (w/w), 21%(w/w), 22%(w/w), 23 % (w/w), 24% (w/w), 25%(w/w), 26%(w/w) or any concentration in-between.

In some cases, the protein dissolving temperatures are kept about 40 °C, about 50 °C, about 60 °C, about 70 °C, about 80 °C, or any temperature in-between.

In some cases, the concentrations of overall polysaccharide dissolved in the above mixtures are about 5% (w/w), 6% (w/w), 7% (w/w), 8% (w/w), 9% (w/w), 10% (w/w), 11% (w/w), 12% (w/w), 13% (w/w), 14% (w/w), 15% (w/w) or any concentration in-between.

In some cases, the concentrations of overall carbohydrate dissolved in a final recipe are about 1% (w/w), 2% (w/w), 3% (w/w), 4% (w/w), 5% (w/w) or any concentration in-between.

In some cases, an additive agent may or may not be added in the final recipe.

In some cases, the concentration of overall additive agent dissolved in the final recipe is 1% (w/w), 1.5% (w/w), 2% (w/w), 3% (w/w), 4% (w/w), 5% (w/w), or any concentration in-between.

The solvents include water, alcohols, ionic compounds, acetic acid, and formic acid.

The final mixture is spun in a HaloSpin™ apparatus for producing polymer fibers to obtain a microfiber scaffold.

The fiber diameter and thickness of the scaffolds may be adjusted by changing the process parameters, concentration of final recipe or combination thereof.

In some cases, the microfiber diameters and porosity may be tuned by changing the process parameters, concentration of final recipe or combination thereof.

In some cases, the position of needles are altered to obtain either randomly or aligned fibers in a microfibrous scaffold and maintain the thickness as stated earlier.

In some cases, the porosity may be indirectly tuned by changing: compositions of final recipe, HaloSpin™ process parameters, thickness, and diameter of the fibers, or combinations thereof.

In some cases, the thickness, area density, and porosity of the scaffold is changed by adjusting the air volume in the range of 20 $m^3$/hour to 35 $m^3$/hour or any magnitude in-between.

In some cases, the thickness, area density, and porosity of the scaffold is changed by adjusting the temperature of air during spinning in the range of 30 °C to 80 °C or any temperature in-between.

In some cases, the thickness, area density, and porosity of the scaffold is changed by adjusting the solution pressure in the range of 0.1 bar to 1.5 bar or any magnitude in-between.

In some cases, the thickness, area density, and porosity of the scaffold is changed by adjusting the temperature of the final recipe during spinning in the range of 30 °C to 80 °C or any temperature in-between.

In some cases, the thickness, area density, and porosity of the scaffold is changed by adjusting the collection belt speed during spinning in the range of 2.3m/hour to 250m/hour or any speed in-between.

The scaffolds are then put in an oven at a certain temperature and duration to get a crosslinked scaffold.

In some cases, the oven temperatures are kept about 121 °C, 130 °C, 140 °C, 145 °C, 150 °C, 155 °C, 160 °C, 165 °C, 170 °C, 175 °C, 180 °C, 190 °C, 200 °C or any temperatures in-between.

In some cases, the scaffolds are kept in the oven for a time duration of about 2 minutes, 45 min, 1 hour, 1.5 hours, 2 hours, or any time frame in-between.

In some cases, the two recipes can be spun together to obtain a bi-layer, tri-layer, or hybrid scaffold. More specifically, the cell culture scaffolds according to the embodiments of the present disclosure may be bi-layer, tri-layer, or hybrid containing fibers from one or more homogeneous solution. This enables to create scaffolds with specific properties to meet diverse cell culture requirements. Bi-layer and tri-layer scaffolds can provide gradients in mechanical strength, porosity, and nutrient diffusion, which are essential for supporting the growth and differentiation of different cell types within a single scaffold. Hybrid scaffolds, containing fibers from multiple homogeneous solutions, offer a combination of material properties, enhancing the scaffold's versatility and functionality. This provides improved cell growth environments, better mimicry of natural tissue structures, and the ability to support complex tissue engineering applications. This multi-layer and hybrid approach ensures the scaffolds can be optimized for high cell yield, structural integrity, and scalability, making them suitable for a wide range of cultured meat production processes.

In some cases, the bi-layer scaffold consists of scaffolds from two recipes that are layered on top of each other. This scaffold is a single embodiment of two different layers.

In some cases, the tri-layer scaffold consists of scaffold from recipe one in the center and scaffolds from recipe two in top and bottom or vice versa. This scaffold is a single embodiment of three different layers.

In some cases, the hybrid scaffold consists of two or more entangled fibers from two or more recipes. This scaffold is a single embodiment of two or more recipes where the fibers from two or more recipes are simultaneously spun to achieve profound entanglement of the fibers within the scaffold structure.

In certain embodiments, a spinning solution is prepared with one plant protein, one polysaccharide, and one carbohydrate, dissolved in one solvent. Example, a recipe of potato scaffold (Figure 2a and Figure 2b) contains Potato protein isolate, pullulan, and dextrose.

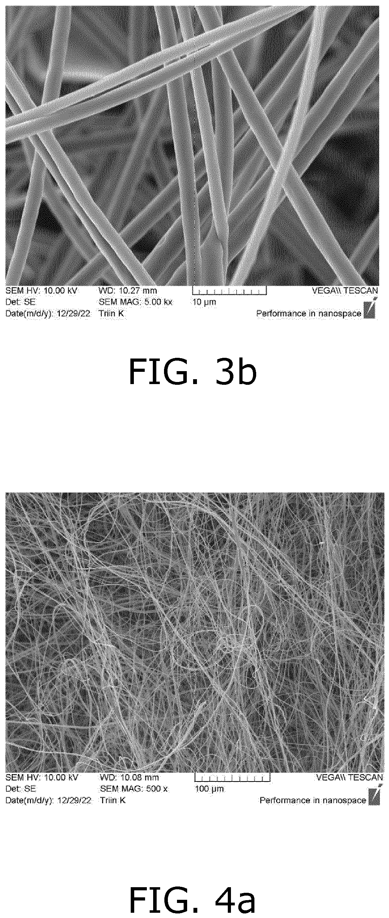

In certain embodiments, a spinning solution is prepared with one plant protein, two polysaccharides, and one carbohydrate, dissolved in one solvent. Example, a recipe of Chitosan-Lupin scaffold (Figure 3a and Figure 3b) contains Lupin protein isolate, chitosan derived from mushroom, pullulan, and dextrose.

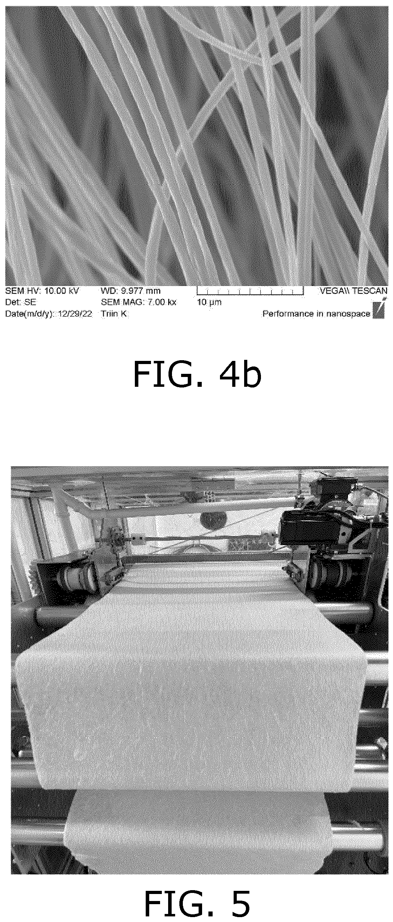

In certain embodiments, a spinning solution is prepared with two or more plant proteins, one polysaccharide, and one carbohydrate, dissolved in one solvent. Example, a recipe of Soy-potato (Figure 4a and Figure 4b) scaffold contains Potato protein isolate, soy protein isolate, pullulan, and dextrose.

In certain embodiments, the concentration of formic acid as a solvent is minimum 1% (w/w) or maximum 85% (w/w) or any concentration in-between.

In certain embodiments, the concentration of acetic acid as a solvent is minimum 1% (w/w) or maximum 90% (w/w) or any concentration in-between.

In certain embodiments, the concentration of alcohol as a solvent is minimum 1% (w/w) or maximum 70% (w/w) or any concentration in-between.

In certain embodiments, the concentration of ionic solutions as a solvent is minimum 1% (w/w) or maximum 10% (wt/wt) or any concentration in-between.

## Scaffold properties and analysis

The microfibrous scaffolds obtained from any final recipe were analyzed including thickness, fiber diameter, area density, morphology in SEM, swelling and degradation in Phosphate buffer saline (PBS), and tensile test.

In certain embodiments, the diameter of individual fibers of the microfibrous scaffold may range up to 0.3 $\mu$m, upto 0.4 $\mu$m, upto 0.5 $\mu$m, upto 0.6, upto 0.7 $\mu$m, upto 0.8 $\mu$m, 0.9 $\mu$m, upto 1 $\mu$m, upto 2 $\mu$m, upto 3 $\mu$m or any ranges in-between and combination thereof.

In certain embodiments, the thickness of the scaffolds can be tuned and obtained about 50 $\mu$m, 100 $\mu$m, 200 $\mu$m, 300 $\mu$m, 400 $\mu$m, 500 $\mu$m, 600 $\mu$m, 700 $\mu$m, 800 $\mu$m, 900 $\mu$m, 1 mm, 5 mm, 1 cm, 3 cm, or any dimensions in-between.

Area density is a unit of measurement used to express the weight of microfibers per unit area of the scaffold, $g/m^2$ (grams per square meter). In certain embodiments, the area density of the obtained scaffolds is in the range of 2 $g/m^2$ to 200 $g/m^2$ or any magnitude in-between. More specifically, according to the embodiments, the area density of the cell culture scaffold may be 2 $g/m^2$ - 200 $g/m^2$. The area density of the cell culture scaffold can thus be from 2, 3, 4, 5, 6, 7, 8, 9, 10, 12, 14, 16, 18, 20, 25, 30, 35, 40, 45, 50, 55, 60, 65, 70, 75, 80, 85, 90, 95, 100, 110, 120, 130, 140, 150, 160, 170, 180, or 190 $g/m^2$ up to 3, 4, 5, 6, 7, 8, 9, 10, 12, 14, 16, 18, 20, 25, 30, 35, 40, 45, 50, 55, 60, 65, 70, 75, 80, 85, 90, 95, 100, 110, 120, 130, 140, 150, 160, 170, 180, 190 or 200 $g/m^2$. This area density range of the cell culture scaffold enables to provide a cell culture scaffold with optimal material distribution and mechanical properties. The cell culture scaffolds having lower area density, e.g., 2 $g/m^2$ - 50 $g/m^2$, are lightweight and highly porous, promoting efficient nutrient diffusion and waste removal, which are crucial for cell viability and growth. The middle range, from 50 $g/m^2$ to 150 $g/m^2$, provides a balanced combination of porosity and mechanical strength, making it suitable for supporting a variety of tissue types while maintaining efficient nutrient transport and waste removal. The higher end of the range (150 $g/m^2$-200 $g/m^2$) offers increased mechanical strength and structural integrity, supporting more complex and larger tissue constructs. This enables to fine-tune the scaffold's physical properties to meet specific requirements for different cultured meat applications, enhancing cell attachment, proliferation, and differentiation. This range of area density ensures that the scaffold can support high cell yields and maintain its structural integrity during large-scale production, making it suitable for diverse and scalable cultured meat production processes.

The morphological explanation of the scaffold from SEM images (Figures 2a, 2b, 3a, 3b, 4a, 4b) is illustrated above.

## Swelling, Degradation and pH test

The microfibrous scaffold's swelling test was conducted in PBS buffer.

The dry scaffolds were soaked in PBS buffer solution (pH 7.4) for a period of 24 hours. The uptake/absorption of PBS was measured gravimetrically using a weighing balance. The ratio of the weight of solution absorbed by the scaffolds to the weight of the dry scaffolds by itself was expressed in percentage as % swelling (Equation 1). Here, $W_d$ is the initial weight of the dry scaffold, and $W_w$ is the weight of the fully swelled scaffold after 24 hours.

$$\text{Equation 1: } \%Swelling = (W_w - W_d)/W_d \times 100$$

The degradation behavior measures the change in sample weight over time under simulated physiological conditions.

The initial weight of scaffolds under dry condition was determined ($W_i$) and the samples were immersed in PBS and incubated at a temperature of 37 °C, 5% $CO_2$. Every two days interval, the buffer was replaced with the fresh PBS buffer, simultaneously the pH of the spent PBS was measured to ascertain the pH drift due to scaffold degradation. The change in pH also determines the presence of solvent residues in case of acidic solvents such as acetic acid and formic acid.

The experiment continued up to 28 days thereafter, the scaffold was removed from the degradation medium, and was rinsed thoroughly with water. The sample was then dried in a hot air oven at 40 °C until the scaffold dried completely. The weight of the dried scaffold was recorded ($W_f$). Percentage degradation, that is the weight loss was estimated from Equation 2.

$$\text{Equation 2: \%Degradation} = (W_i - W_f)/W_i \times 100$$

In certain embodiments, swelling of a microfibrous scaffold is in the range of 1000% to 5500% or any magnitude in-between. More specifically, according to the embodiments, the swelling of the cell culture scaffold is 1000% - 5500%. The swelling of the cell culture scaffold can thus be from 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, or 5400 % up to 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400 or 5500 %.

In certain embodiments, the swelling of any microfibrous scaffold can be adjusted by changing the fiber diameter, area density, thickness, concentration of final solution, crosslinking temperature, crosslinking time, or combinations thereof.

In certain embodiments, the swelling of any microfibrous scaffold can be tuned by adding more than one plant protein.

In certain embodiments, the swelling of any microfibrous scaffolds can be tuned by adding more than one polysaccharide.

In certain embodiments, the swelling of any microfibrous scaffolds can be tuned by adding more than one carbohydrate.

Hydrophilic amino acids, such as serine, threonine, and asparagine, are commonly found in plant proteins and contribute to their water-binding capacity. This hydrophilicity enables the microfibrous scaffold to absorb and retain water, creating a hydrated microenvironment for cultured meat cells. Swelling is crucial for promoting nutrient diffusion, cell attachment, and metabolic activities within the cultured meat construct. By adjusting the scaffold's composition and incorporating different plant proteins, it will tailor the swelling behavior to achieve optimal nutrient transport and support cell growth.

The polysaccharides used in scaffolds, such as pullulan, alginate and starch, are hydrophilic in nature, allowing them to readily absorb and retain water. This property contributes to the scaffold's ability to swell when exposed to aqueous solutions, such as cell culture media or physiological fluids. Proper swelling is crucial for creating a hydrated and supportive environment for cultured meat cells. It enables efficient nutrient and oxygen transport to the cells, promoting their growth and proliferation. Controlled swelling also assists in maintaining the structural integrity of the scaffold during tissue maturation. By adjusting the polysaccharide composition and crosslinking density, it will regulate the swelling behavior to achieve an optimal balance between hydration and mechanical stability.

In certain embodiments, the degradation of a microfibrous scaffold after 28 days is in the range of 10% to 70% or any combinations in-between. More specifically, according to the embodiments, the degradation of the cell culture scaffold may be 10 % - 70 % after 28 days in phosphate buffer saline. The degradation of the cell culture scaffold can thus be from 10, 15, 20, 25, 30, 35, 40, 45, 50, 55, 60, or 65 % up to 15, 20, 25, 30, 35, 40, 45, 50, 55, 60, 65, or 70 %.

In certain embodiments, the rate of degradation of any microfibrous scaffold can be adjusted by changing the fiber diameter, area density, thickness, concentration of final solution, crosslinking temperature, crosslinking time, or any combinations thereof.

In certain embodiments, the rate of degradation of any microfibrous scaffolds can be adjusted by adding more than one plant protein.

In certain embodiments, the rate of degradation of any microfibrous scaffolds can be adjusted by adding more than one polysaccharide.

In certain embodiments, the rate of degradation of any microfibrous scaffolds can be adjusted by adding more than one carbohydrate.

The degradation behavior of plant protein-based scaffolds in PBS can be tailored by adjusting factors such as the composition of the scaffold, the crosslinking density, and the presence of specific functional groups. The different plant proteins like potato, soy, pea, lupin, canola, fava, mung, corn, wheat, rice, barley, sunflower have specific function groups that can lead to faster degradation. These factors can influence the scaffold's susceptibility to hydrolytic degradation in PBS and allow it to control the degradation rate as needed for cultured meat production. Plant proteins, such as soy and pea proteins, can undergo enzymatic degradation, breaking down into biocompatible and biodegradable components. The presence of functional groups like amino and carboxyl groups in plant proteins enhances their susceptibility to enzymatic degradation, enabling the scaffold to gradually assimilate into the cultured meat tissue over time.

The biodegradability of polysaccharides like alginate and mushroom chitosan is advantageous in cultured meat production. These polymers can be broken down through enzymatic or hydrolytic processes, gradually degrading over time as the cultured meat tissue matures. Controlled degradation is vital in tissue engineering to ensure that the scaffold integrates with the developing tissue and does not hinder tissue remodeling. The ability to tailor the degradation rate allows for the scaffold's gradual assimilation into the surrounding tissue, eventually leaving behind only the cultured meat tissue without any remaining scaffold remnants. Proper degradation kinetics will also contribute to reducing the risk of inflammation or adverse reactions within the cultured meat construct.

During the scaffold swelling test, pH of the buffer media was also analyzed after 24 hours, to confirm any changes due to fabrication of microfibers and presence of organic acids such as acetic acid or formic acid.

In certain embodiments, the pH of the buffer media after 24 hours for any microfibrous scaffolds is seen in the range of 6.8 to 7.4 or any value in-between. This indicates no significant change in the scaffolds due to residue of organic solvents, or fabrication of fibers, or crosslinking.

**Tensile test**

The scaffolds' strength was evaluated utilizing a benchtop Universal Testing Machine (UTM). Tensile tests were conducted following the ASTM D638 standard, involving the scaffolds being cut into a dumbbell shape for the testing procedure. The scaffolds were stretched until the specimen broke. The force vs displacement was recorded and later analyzed for Young's modulus, ultimate stress, and elongation of the scaffolds.

The tensile strength was conducted on the dry, crosslinked scaffolds.

In certain embodiments, the Young's modulus of a scaffold is in the range of 500 kPa to 6500 kPa or any value in-between. The Young's modulus of the scaffold can be from 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300 or 6400 kPa up to 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400 or 6500 kPa.

In certain embodiments, the Young's modulus of a scaffold can be adjusted by changing the fiber orientation, fiber diameter, area density, concentration of final solution, crosslinking temperature, crosslinking time, or any combinations thereof.

In certain embodiments, the young's modulus of a scaffold can be adjusted by adding more than one plant protein.

In certain embodiments, the young's modulus of a scaffold can be adjusted by adding more than one polysaccharide.

In certain embodiments, the young's modulus of a scaffold can be adjusted by adding more than one carbohydrate.

In certain embodiments, the ultimate stress of a scaffold is in the range of 20kPa to 900kPa or any value in-between.

In certain embodiments, the ultimate stress of a scaffold can be adjusted by changing the fiber orientation, fiber diameter, area density, concentration of final solution, crosslinking temperature, crosslinking time, or any combinations thereof.

In certain embodiments, the ultimate stress of a scaffold can be adjusted by adding more than one plant protein.

In certain embodiments, the ultimate stress of a scaffold can be adjusted by adding more than one polysaccharide.

In certain embodiments, the ultimate stress of a scaffold can be adjusted by adding more than one carbohydrate.

In certain embodiments, the % elongation of a scaffold is in the range of 1% to 20% or any value in-between.

In certain embodiments, the % elongation of a scaffold can be adjusted by changing the fiber orientation, fiber diameter, area density, concentration of final solution, crosslinking temperature, crosslinking time, or any combinations thereof.

In certain embodiments, the % elongation of a scaffold can be adjusted by adding more than one plant protein.

In certain embodiments, the % elongation of a scaffold can be adjusted by adding more than one polysaccharide.

In certain embodiments, the % elongation of a scaffold can be adjusted by adding more than one carbohydrate.

The structural strength of the scaffolds varies upon the presence of proteins in the scaffolds. The lupin protein and potato protein showed higher young's modulus and ultimate stress due to the stronger molecular bonding compared to soy protein. The lupin protein and potato protein contain more cellulosic fibers compared to the soy proteins which make lupin stronger compared to soy. Whereas, the soy protein is always preferred over the lupin protein due to its complete amino acid profile. Therefore, it may be stated that a combination of soy and potato protein in a single embodiment is desirable where the potato will give more structural strength and the soy will be beneficial for human diet.

The tensile properties are influenced by the structural characteristics of these proteins in the scaffold. For example, soy protein contains a high proportion of $\beta$-sheets and $\alpha$-helices, imparting good tensile strength and stability to the scaffold. In contrast, corn and wheat proteins are rich in gliadin and glutenin, which contribute to the viscoelastic behavior and extensibility of the scaffold. The specific combination and ratios of these proteins in the scaffold formulation dictate its overall tensile performance.

Incorporating polysaccharides such as pullulan, starch, alginate, and mushroom chitosan into a scaffold for cultured meat significantly impacts its tensile properties. The choice of polysaccharide in a final recipe influences the scaffold's mechanical behavior, including modulus, ultimate stress, and elongation. Polysaccharides like pullulan and alginate contribute to a higher modulus, providing greater stiffness and structural support to the scaffold. This enhanced rigidity is beneficial for maintaining the scaffold's shape and preventing deformation during cultured meat tissue development. On the other hand, mushroom chitosan can increase the scaffold's ultimate stress due to its excellent mechanical strength and resilience, enabling the scaffold to withstand higher mechanical loads. Additionally, polysaccharides like starch and alginate offer good elongation properties, allowing the scaffold to stretch and deform without breaking. This characteristic prevents premature failure and ensures the scaffold's ability to withstand handling and mechanical forces during the cultured meat production process.

Furthermore, these polysaccharides possess functional groups that interact with proteins, promoting binding and affecting the scaffold's tensile properties. For instance, the hydroxyl groups in pullulan, starch, and alginate can form hydrogen bonds with proteins, enhancing the scaffold's compatibility with cultured meat cells and promoting cell adhesion. Mushroom chitosan, with its amino and hydroxyl groups, can participate in ionic interactions and hydrogen bonding with proteins, further enhancing protein-scaffold interactions and strengthening the scaffold's mechanical properties. These advantageous functional groups create a favorable microenvironment within the scaffold, promoting the adhesion, proliferation, and differentiation of cultured meat cells. As a result, the tailored interaction between the polysaccharide-based scaffold and proteins contributes to the overall tensile properties and biocompatibility of the scaffold, making it a suitable platform for the successful production of cultured meat.

Plant proteins possess various functional groups, such as hydroxyl, carboxyl, amino, and sulfhydryl groups, which contribute to their interactions with polysaccharides and their surroundings. The crosslinking degree with carbohydrates consumes the amino groups and thereby controls the overall scaffold strength.

### Cell Culture

The type of cells and cell lines that can be grown on these innovative microfibrous scaffold include, but are not limited to, myoblasts, adult stem cells, adipocytes, embryonic stem cells, mesenchymal stem cells, pluripotent stem cells, primary muscle cells, muscle satellite cells, fibroblasts, fat stem cells, muscle side population cells, and any relevant cell line for cultivating meat. Cells from chicken, pig, bovine, fish, or other species may be utilized to grow meat on any microfibrous scaffolds. The cells can be seeded at a density of 50,000, 80,000, 100,000, 200,000, 500,000 or more cells/cm$^2$, or any range of density in-between. The cells are seeded to proliferate and form tissues like myotubes. The cells can be cultured until the scaffolds degrade more than 80%. The growth of cells on the scaffolds may be in the form of individual cells confluence in the range of 40% to 80% or any magnitude in-between.

The amount of biomass production of cells in the scaffolds may be weighted from 40% to 70% or any weight in between. However, the rest of the part in the scaffolds may be present or may not be present depending upon the degradation of the scaffold. The scaffold containing cells may be or may not be differentiated in the final product. The scaffold has the potential to be used in any bioreactor. Where the scaffolds can be fixed, seeded with cells, and cultured until the cells are differentiated.

### Scalability

The HaloSpin™ apparatus is utilized to produce microfibrous scaffolds (Figure 5) from any final recipe. The production capacity of microfibrous scaffolds is obtained in the range of 0.1 kg/hour to 25 kg/hour or any capacity in-between. The production capacity is adjusted by adding more needles, increasing the belt speed and width, changing the concentration of the final recipe, or any combinations thereof. The production duration may be increased up to 30 days, continuously. The width of microfibrous scaffolds may be adjusted by increasing the width of the substrate where the fibers are collected. The width of the substrate is in the range of 30 cm to 300 cm or any value in-between.

In an embodiment, the present disclosure provides a cell culture scaffold comprising plant-based microfibrous in a 3D structure, wherein the plant-based microfibrous comprise at least 60% of one or more plant-based proteins, one or more polysaccharide, one or more carbohydrates.

In different embodiments of the cell culture scaffold, the parameters of the cell culture scaffold may be following. A diameter of the plant-based microfibrous may be 0.3 $\mu$m - 3 $\mu$m. A thickness of the cell culture scaffold may be 100 $\mu$m - 3 cm. A thickness of the cell culture membrane may be 50 $\mu$m - 100 $\mu$m. An area density may be 2 g/m$^2$ - 200 g/m2. A swelling may be 1000% - 5500%. A degradation may be 10% - 70% after 4 weeks in phosphate buffer saline.

Therein the one or more plant-based proteins may be selected from a plant protein group comprising soy protein, pea protein, lupin protein, canola protein, potato protein, fava protein, mung protein, corn protein, wheat protein, rice protein, barley protein, sunflower protein and combinations or derivatives thereof. The polysaccharide may comprise pullulan, starch, pectin, alginate, chitosan and combinations or derivatives thereof. Carbohydrates may be selected from a carbohydrate group comprising dextrose, fructose, galactose, glyceraldehyde, glucose, isomalt, lactitol, maltose, mannitol, maltitol, ribose, sorbitol, xylitol, xylose.

The fibrous scaffold architecture may create a 3D network resembling a sheet-like, entangled fibers in a single embodiment, allowing cell interactions, nutrient diffusion, and waste removal. Adjusting process parameters enables better control over fiber diameter and scaffold thickness, enhancing uniform porosities and continuous fiber orientation and entanglement.

In another embodiment, the present disclosure provides a method for making a cell culture scaffold comprising plant-based microfibrous in a 3D structure, wherein the method comprises dissolving one or more plant-based proteins in a solvent, adding one or more polysaccharide and one or more carbohydrates until the solution becomes homogeneous, spinning the microfibrous from the homogeneous solution by a combined forces of air volume and centrifugal forces pushing fibers out from one or more spinning needles, obtaining the cell culture scaffold from the spun microfibrous by collecting the spun fibers on a substrate, thermal processing the obtained cell culture scaffold.

The homogenous solution may comprise 8% w/w - 26 % w/w of one or more proteins, 5% w/w - 15% w/w of one or more polysaccharide, 1% w/w - 5% w/w of one or more carbohydrates. The parameters of spinning may be: an air volume 20 m$^3$/hour - 35 m$^3$/hour, an air temperature 35 °C - 80 °C, solution pressure 0.1 bar - 1.5 bar, wherein the substrate is rotating at a speed rate of 2.3 m/hour - 250 m/hour. The the heating of the obtained cell culture scaffold may be performed by keeping the obtained cell culture scaffold in a hot air at 121°C - 200 °C for 20 - 120 minutes. The scaffolds can be bi-layer, tri-layer, or hybrid contains fibers from one or more homogenous solution. The production of scaffolds may be about 0.1 kg/hour - 25 kg/hour in a HaloSpin™ apparatus. The continuous spinning of any recipe may increase up to 30 days.

### Example 1: Protein-based microfiber scaffold.

Potato protein-based scaffold was produced, containing 70% potato protein isolate, 22% pullulan, and 8% dextrose in the scaffold. The recipe was prepared by dissolving potato protein isolate, pullulan, and dextrose in distilled water. The solution was kept mixing for 2-3 days to achieve complete dissolution. The overall concentrations in the solution were: potato protein - 18%, pullulan - 10%, and dextrose - 2%. The solution was spun in a device for producing polymer fibers through a 19-gauge needle. The process parameters were maintained as follows: number of needles - 8, solution pressure - 0.61 bar, air volume - 25 m$^3$/hour, air temperature - 30 °C, ambient temperature - 24 °C, relative humidity - 22%, and collection speed - 20 m/hour, producing a scaffold of thickness and area density corresponds to 0.5 mm and 32 g/m$^2$ respectively. The scaffold was then placed in a hot oven at 170 °C for 2 hours to crosslink the microfibers.

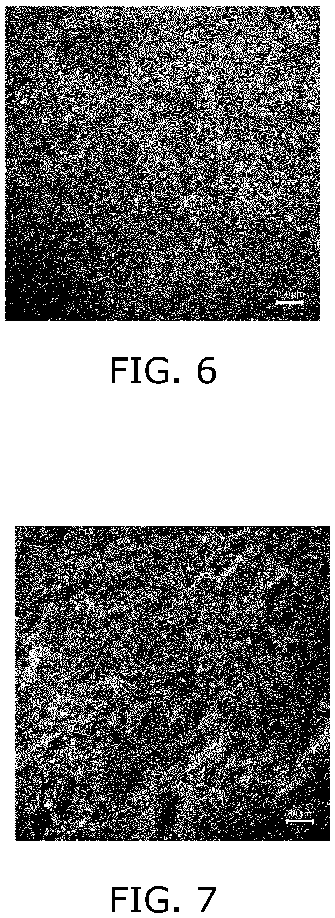

The SEM images (Figures 2a and 2b) reveal a network of microfibers that closely mimic the ECM found in tissues, making them highly beneficial for 3D cell culture. The scaffold's swelling capacity was 3000% after 24 hours, and its degradation was 34% after 28 days in Phosphate-Buffered Saline (PBS), indicating a high ability to absorb culture media, which maintains cell hydration and provides an environment suitable for cell growth. This porous structure facilitates efficient nutrient and oxygen diffusion, crucial for cell viability and proliferation, and the degradation behavior aligns well with the cultivation duration of cultured meat, ensuring scaffold integrity during the growth phase. Additionally, the mechanical properties of the scaffold, including a Young's modulus of 3400 kPa, ultimate stress of 180 kPa, and elongation of 15%, demonstrate sufficient strength and flexibility to support 3D cell culture and differentiation, essential for the growth and differentiation of muscle cells needed to create structured meat products. The scaffold's biocompatibility was tested with myoblast cells (C2C12), showing capabilities such as adhesion, proliferation, and differentiation, as illustrated by the live cells (stained with calcein) in the scaffold after 7 days of proliferation (Figure 6). Moreover, the use of potato protein enhances the nutritional value of the cultured meat by providing essential amino acids and other nutrients, and being derived from edible plant sources, the scaffold itself contributes to the overall edibility and safety of the final product, making it a suitable and sustainable choice for cultured meat production.

### Example 2: Protein-Polysaccharide-based scaffold.

A Chitosan-Lupin based scaffold consists of 40% lupin protein isolate, 40% chitosan derived from mushrooms, 12% pullulan, and 8% dextrose. The inclusion of chitosan, being a polysaccharide, enhances the scaffold's antimicrobial properties, creating a sterile environment conducive to cell culture. Additionally, lupin protein, derived from an edible plant source, boosts the nutritional value of the cultured meat by providing essential amino acids and other nutrients. This combination also makes the scaffold safe, edible, and sustainable choice for cultured meat production. The use of chitosan and lupin protein ensures the scaffold's strength and flexibility, while also supporting the growth and differentiation of cells.

The materials used included lupin protein isolate, chitosan, food-grade pullulan, and dextrose. These components were dissolved in formic acid to create the solution with the following concentrations: 8.5% lupin protein, 7.5% chitosan, 3.2% pullulan, and 1.6% dextrose. The solution was then spun using a device for producing polymer fibers equipped with 21-gauge needles. The process parameters included 16 needles, solution pressure of 2.5 bar, air volume of 29 $m^3$/hour, air temperature of 45 °C, ambient temperature of 24 °C, relative humidity of 20%, and a collection speed of 25 m/hour. This process produced a scaffold with a thickness of 0.5 mm and an area density of 35 $g/m^2$. The scaffold was subsequently placed in an oven at 170 °C for 2 hours to crosslink the microfibers. SEM images (Figures 3a and Figure 3b) displayed a highly porous structure and similar properties as explained in the previous example. Also, the scaffold demonstrated a swelling capacity of 2800% after 24 hours and degraded by 40% after 28 days in PBS. These properties are comparable to those in the previous example, but with slightly improved mechanical properties: a Young's modulus of 4000 kPa, ultimate stress of 240 kPa, and elongation of 20%. Biocompatibility tests with myoblast cells (C2C12) confirmed the scaffold's ability to support cell adhesion, proliferation, and differentiation, as shown by the presence of live cells stained with calcein after 7 days of proliferation (Figure 7).

DETAILED DESCRIPTION OF THE DRAWINGS

Figure 1 presents a section of a plant-based scaffold with a thickness of 500 $\mu$m, showcasing embedded microfibers that form a 3D network resembling a felt-like sheet. This network is composed of entangled fibers with a narrow distribution of fiber diameters, providing the appropriate thickness and fiber orientation (random or aligned) essential for supporting cell interactions, nutrient diffusion, and waste removal. The distinct structure of this scaffold closely mimics the natural extracellular matrix (ECM), which is crucial for cell growth and tissue development.

Figure 2a provides a scanning electron microscopy (SEM) image at 500x magnification of a microfibrous scaffold composed of 60% potato protein isolate, 32% pullulan, and 8% dextrose. The image illustrates the scaffold's microfibrous architecture, which closely resembles the ECM, promoting high porosity, a high surface-to-volume ratio, and free pathways for nutrient absorption and exchange. The image specifications are SEM HV 10.00 kV, Det: SE, WD: 10.18 mm, SEG MAG: 500x, at a scale of 100 micrometers. These randomly oriented fibers create a highly porous structure, facilitating efficient cell proliferation and differentiation.

Figure 2b shows a SEM image of the same scaffold at a higher magnification of 7000x. This image highlights the finer details of the fiber network, revealing the small gaps between fibers that form a 3D interconnected network. The image specifications are SEM HV 10.00 kV, Det: SE, WD: 10.18 mm, SEG MAG: 7.00 kx, at a scale of 10 micrometers. This interconnectivity is suggested by the gaps in the adjacent planes, indicating connections between the layers. The scaffold's structure supports efficient nutrient diffusion and waste removal, essential for maintaining cell viability and promoting tissue maturation.

Figure 3a presents a SEM image of a microfibrous scaffold at 500x magnification, composed of 66% chitosan from mushrooms, 14% lupin protein isolate, 15% pullulan, and 5% dextrose. The image highlights the scaffold's highly porous structure and fiber entanglement, which are critical for mimicking the ECM. The image specifications are SEM HV 10.00 kV, Det: SE, WD: 10.26 mm, SEG MAG: 500x, at a scale of 100 micrometers. The inclusion of chitosan, a polysaccharide, enhances the scaffold's antimicrobial properties, creating a sterile environment conducive to cell growth and differentiation.

Figure 3b offers a closer view of the scaffold at 5000x magnification, revealing the intricate fiber network in greater detail. The small gaps between fibers suggest the scaffold's 3D interconnected network, vital for nutrient diffusion and cell interactions. The image specifications are SEM HV 10.00 kV, Det: SE, WD: 10.27 mm, SEG MAG: 5.00 kx, at a scale of 10 micrometers. The scaffold demonstrates a swelling capacity of 2800% after 24 hours and degrades by 40% after 28 days in Phosphate-Buffered Saline (PBS), indicating its ability to maintain structural integrity while providing a supportive environment for cell growth and tissue formation.

Figure 4a provides a SEM image at 500x magnification of a microfibrous scaffold consisting of 66% soy protein isolate, 14% potato protein isolate, 16% pullulan, and 4% glucose. The image shows the scaffold's microfibrous architecture, with randomly oriented fibers that create a highly porous structure. The image specifications are SEM HV 10.00 kV, Det: SE, WD: 10.08 mm, SEG MAG: 500x, at a scale of 100 micrometers. This scaffold is designed to support cell adhesion, proliferation, and differentiation, closely mimicking the ECM found in natural tissues.

Figure 4b presents a detailed SEM image at 7000x magnification of the same scaffold as Figure 4a. This image reveals the small gaps between fibers, contributing to the scaffold's 3D interconnected network, which facilitates nutrient absorption and exchange. The image specifications are SEM HV 10.00 kV, Det: SE, WD: 9.977 mm, SEG MAG: 7.00 kx, at a scale of 10 micrometers. The scaffold's structure is optimized for high cell yield, making it suitable for large-scale cultured meat production.

Figure 5 illustrates the continuous production of microfibrous scaffolds using the device for producing polymer fibers. This image emphasizes the scalability and efficiency of the production process, which can produce scaffolds with controlled fiber diameters, thicknesses, and area densities. The apparatus allows for the mass production of scaffolds, supporting the growing demand for sustainable and ethical cultured meat production.

Figure 6 shows C2C12 myoblast cells after 7 days of proliferation on a potato protein-based scaffold. The live cells, stained with calcein, are visibly attached and spread across the scaffold, demonstrating the scaffold's biocompatibility and ability to support cellular growth, adhesion, and differentiation. The image is at a scale of 100 micrometers. This scaffold contributes to the overall edibility and safety of the final cultured meat product by providing essential amino acids and nutrients.

Figure 7 presents C2C12 myoblast cells after 7 days of proliferation on a chitosan-lupin-based scaffold. The cells are actively proliferating, as indicated by the calcein staining, confirming the scaffold's suitability for supporting cell adhesion, growth, and differentiation. The image is at a scale of 100 micrometers. The scaffold's antimicrobial properties, derived from chitosan, further enhance its potential for use in sterile, large-scale cultured meat production.

## Claims

1. A cell culture scaffold comprising plant-based microfibrous in a 3D structure, wherein the plant-based microfibrous comprise

   - at least 60% of one or more plant-based proteins,
   - one or more polysaccharides,
   - one or more carbohydrates.

2. The cell culture scaffold according to claim 1, wherein a diameter of the plant-based microfibrous is 0.3 $\mu$m - 3 $\mu$m.

3. The cell culture scaffold according to claim 1 or 2, wherein a thickness of the cell culture scaffold 50 $\mu$m -30 000 $\mu$m.

4. The cell culture scaffold according to claim 3, wherein a thickness of the cell culture scaffold is 50 $\mu$m - 100 $\mu$m.

5. The cell culture scaffold according to any of the preceding claims, wherein an area density of the cell culture scaffold is 2 g/m$^2$ - 200 g/m$^2$.

6. The cell culture scaffold according to any of the preceding claims, wherein a swelling of the cell culture scaffold is 1000% - 5500%.

7. The cell culture scaffold according to any of the preceding claims, wherein a degradation of the cell culture scaffold is 10% - 70% after 28 days in phosphate buffer saline.

8. The cell culture scaffold according to any of the preceding claims, wherein the one or more plant-based proteins are selected from a plant protein group comprising potato protein, soy protein, pea protein, lupin protein, canola protein, fava protein, mung protein, corn protein, wheat protein, rice protein, barley protein, or sunflower protein or combinations or derivatives thereof.

9. The cell culture scaffold according to any of the preceding claims, wherein the one or more polysaccharides are selected from a group comprising pullulan, starch, pectin, alginate, chitosan, or mushroom chitosan or combinations or derivatives thereof.

10. The cell culture scaffold according to any of the preceding claims, wherein the one or more carbohydrates are selected from a carbohydrate group comprising dextrose, fructose, galactose, glyceraldehyde, glucose, isomalt, lactitol, maltose, mannitol, maltitol, ribose, sorbitol, xylitol, or xylose or combinations thereof.

11. A method for making a cell culture scaffold comprising plant-based microfibrous in a 3D structure, wherein the method comprises

    - dissolving one or more plant-based proteins in a solvent,

- adding one or more polysaccharides and one or more carbohydrates until the solution becomes a homogeneous solution,
- spinning the plant-based microfibrous from the homogeneous solution by a combined forces of air volume and centrifugal forces pushing the homogeneous solution out from one or more spinning needles,
- obtaining the cell culture scaffold from the spun plant-based microfibrous by collecting the spun plant-based microfibrous on a substrate,
- heating the obtained cell culture scaffold.

12. The method according to claim 11, wherein the homogeneous solution comprises

  - 8% w/w - 26 % w/w of one or more plant based proteins,
  - 5% w/w - 15% w/w of one or more polysaccharides,
  - 1% w/w - 5% w/w of one or more carbohydrates.

13. The method according to claim 11, wherein parameters of spinning are: an air volume 20 m$^3$/hour - 35 m$^3$/hour, an air temperature 30 °C - 80 °C, solution pressure 0.1 bar - 1.5 bar, wherein the substrate is rotating at a speed rate of 2.3 m/hour - 250 m/hour.

14. The method according to any of the claims 11-13, wherein the heating of the obtained cell culture scaffold is performed by keeping the obtained cell culture scaffold in a hot air at 121°C - 200 °C for 2 - 120 minutes.

15. The method according to any of the claims 11-14, wherein the cell culture scaffold is bi-layer, tri-layer, or hybrid containing fibers from one or more homogeneous solution.

# FIG. 1

# FIG. 2a

FIG. 2b

FIG. 3a

FIG. 3b

FIG. 4a

SEM HV: 10.00 kV    WD: 9.977 mm                                    VEGA\\ TESCAN
Det: SE             SEM MAG: 7.00 kx    10 µm
Date(m/d/y): 12/29/22    Triin K                     Performance in nanospace

FIG. 4b

FIG. 5

FIG. 6

FIG. 7

Europäisches
Patentamt

European
Patent Office

Office européen
des brevets

# EUROPEAN SEARCH REPORT

**Application Number**

EP 24 19 4353

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/245658 A1 (JOHNSON JED K [US] ET AL) 6 August 2020 (2020-08-06) * claims; examples * | 1,8-10 | INV. C12N5/00 |
| A | WO 2022/249076 A1 (INNOCENT MEAT GMBH [DE]) 1 December 2022 (2022-12-01) | 1-15 | |
| A | XIANG NING ET AL: "3D porous scaffolds from wheat glutenin for cultured meat applications", BIOMATERIALS, ELSEVIER, AMSTERDAM, NL, vol. 285, 27 April 2022 (2022-04-27), XP087061870, ISSN: 0142-9612, DOI: 10.1016/J.BIOMATERIALS.2022.121543 [retrieved on 2022-04-27] | 1-15 | |
| A | CN 116 536 257 A (OCEAN UNIV CHINA ET AL.) 4 August 2023 (2023-08-04) | 1-15 | |
| A,P | WO 2024/059358 A1 (TENDER FOOD INC [US]) 21 March 2024 (2024-03-21) | 1-15 | **TECHNICAL FIELDS SEARCHED (IPC)** |
| A | WO 2022/038240 A2 (MERCK PATENT GMBH [DE]) 24 February 2022 (2022-02-24) | 1-15 | C12N A61L C12M |
| A | LEKO LIN ET AL: "Alimentary 'green' proteins as electrospun scaffolds for skin regenerative engineering : Electrospun soy scaffold for regenerative engineering", JOURNAL OF TISSUE ENGINEERING AND REGENERATIVE MEDICINE, vol. 7, no. 12, 1 December 2013 (2013-12-01), pages 994-1008, XP055384956, US ISSN: 1932-6254, DOI: 10.1002/term.1493 | 1-15 | |

-/--

The present search report has been drawn up for all claims

| Place of search | Date of completion of the search | Examiner |
|---|---|---|
| Munich | 5 February 2025 | Armandola, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

**Application Number**

EP 24 19 4353

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WONGKANYA RATCHADA ET AL: "Electrospinning of alginate/soy protein isolated nanofibers and their release characteristics for biomedical applications", JOURNAL OF SCIENCE: ADVANCED MATERIALS AND DEVICES, vol. 2, no. 3, 1 September 2017 (2017-09-01), pages 309-316, XP093246793, ISSN: 2468-2179, DOI: 10.1016/j.jsamd.2017.05.010 * Materials and Methods; table 1 * | 1-15 | |

- - - - -

**TECHNICAL FIELDS SEARCHED (IPC)**

The present search report has been drawn up for all claims

| Place of search | Date of completion of the search | Examiner |
|---|---|---|
| Munich | 5 February 2025 | Armandola, Elena |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 19 4353

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2025

| Patent document cited in search report | Publication date | Patent family member(s) | | Publication date |
|---|---|---|---|---|
| US 2020245658 A1 | 06-08-2020 | AU | 2020216496 A1 | 08-07-2021 |
| | | BR | 112021012871 A2 | 21-09-2021 |
| | | CA | 3127370 A1 | 06-08-2020 |
| | | EP | 3918117 A1 | 08-12-2021 |
| | | IL | 284948 A | 30-09-2021 |
| | | IL | 316394 A | 01-12-2024 |
| | | JP | 2022523724 A | 26-04-2022 |
| | | SG | 11202108167R A | 30-08-2021 |
| | | US | 2020245658 A1 | 06-08-2020 |
| | | US | 2024284947 A1 | 29-08-2024 |
| | | WO | 2020160533 A1 | 06-08-2020 |
| WO 2022249076 A1 | 01-12-2022 | EP | 4347782 A1 | 10-04-2024 |
| | | WO | 2022249076 A1 | 01-12-2022 |
| CN 116536257 A | 04-08-2023 | NONE | | |
| WO 2024059358 A1 | 21-03-2024 | US | 2024090531 A1 | 21-03-2024 |
| | | WO | 2024059358 A1 | 21-03-2024 |
| WO 2022038240 A2 | 24-02-2022 | AU | 2021328232 A1 | 02-03-2023 |
| | | AU | 2024216368 A1 | 12-09-2024 |
| | | CA | 3190453 A1 | 24-02-2022 |
| | | CN | 116348591 A | 27-06-2023 |
| | | EP | 4199746 A2 | 28-06-2023 |
| | | IL | 300593 A | 01-04-2023 |
| | | JP | 2023539149 A | 13-09-2023 |
| | | KR | 20230037658 A | 16-03-2023 |
| | | US | 2024010983 A1 | 11-01-2024 |
| | | WO | 2022038240 A2 | 24-02-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020016368 W
- US 2019066089 W